Europäisches Patentamt

European Patent Office

Office européen des brevets

(19)

(11) Veröffentlichungsnummer: **0 174 478 B1**

(12) **EUROPÄISCHE PATENTSCHRIFT**

(45) Veröffentlichungstag der Patentschrift: **11.12.91**

(21) Anmeldenummer: **85109471.4**

(22) Anmeldetag: **08.09.82**

(51) Int. Cl.5: **G01N 33/92**

(60) Veröffentlichungsnummer der früheren Anmeldung nach Art. 76 EPÜ: **0 074 610**

(54) Verfahren zur selektiven extrakorporalen Präzipitation von Low Density Lipoproteinen aus Vollserum oder Plasma.

(30) Priorität: **10.09.81 DE 3135814**
**13.05.82 DE 3217925**

(43) Veröffentlichungstag der Anmeldung:
**19.03.86 Patentblatt 86/12**

(45) Bekanntmachung des Hinweises auf die Patenterteilung:
**11.12.91 Patentblatt 91/50**

(84) Benannte Vertragsstaaten:
**AT BE CH DE FR GB IT LI LU NL SE**

(56) Entgegenhaltungen:
**EP-A- 0 035 211**
**DE-A- 2 013 644**
**FR-A- 2 381 311**
**US-A- 4 215 993**

(73) Patentinhaber: **B. Braun-SSC AG**
**Gerliswilstrasse 74**
**CH-6020 Emmenbrücke(CH)**

(72) Erfinder: **Seidel, Dietrich, Prof.**
**Dahlmannstr. 23**
**W-3400 Göttingen(DE)**
Erfinder: **Wieland, Heinrich, Dr.**
**Hagenmatenstr. 29**
**W-7800 Freiburg(DE)**
Erfinder: **Rath, Dieter**
**Franz Gleim-Str. 67**
**W-3508 Melsungen(DE)**
Erfinder: **Rosskopf, Gerhard, Dr.**
**Heiligenberstr. 29**
**W-3501 Fuldabrück-Dörnhagen(DE)**

(74) Vertreter: **von Kreisler, Alek, Dipl.-Chem. et al**
**Deichmannhaus am Hauptbahnhof**
**W-5000 Köln 1(DE)**

## Beschreibung

Die Fortschritte auf dem Gebiet der Analytik des Lipoproteinsystems der letzten Jahre haben ergeben, daß die in älteren epidemiologischen Daten erhobenen engen Korrelationen zwischen Plasmacholesterinkonzentrationen und dem Risiko einer frühzeitigen Atherosklerose, speziell der Koronarsklerose, im wesentlichen durch cholesterinreiche β-Lipoproteine gegeben sind. Im Blut des Menschen finden sich normalerweise ca. 70 - 80% des gesamten Cholesterins an die low density bzw. β-Lipoproteine gebunden, der Rest verteilt sich auf andere Lipoproteinfraktionen (im wesentlichen VLDL, HDL und Chylomikronen) (LDL = Low Density Lipoproteine; VLDL = Very Low Density Lipoproteine; HDL = High Density Lipoproteine). In Krankheitsprozessen, die mit einem gestörten Fettstoffwechsel bzw. erhöhten Plasmalipidkonzentrationen einhergehen und die zur frühzeitigen Atherosklerose führen, kann sich der prozentuale Anteil des LDL- oder β-Cholesterins (LDL = β-Lipoprotein) am Gesamtcholesterin sogar noch weit erhöhen. Das heisst, eine Hypercholesterinämie ist in der Regel durch eine Hyper-β-Lipoproteinämie hervorgerufen. In der Vergangenheit hat es daher auch nicht an Versuchen gefehlt

1. die low density Lipoproteine selektiv zu messen und

2. solche, möglichst selektiv aus der Zirkulation zu eliminieren.

Sowohl das eine als auch das andere ist bisher unter Berücksichtigung des Standes der Technik nicht befriedigend gelöst worden. Die üblichen Verfahren, low density Lipoproteine zu quantifizieren beruhen entweder auf der Verwendung der Trennung des Lipoproteinspektrums in Dichteklassen unter Zuhilfenahme der Ultrazentrifuge oder auf der Trennung des Lipoproteinspektrums im elektrischen Feld, ein Verfahren, das man sich bei der sog. Lipoproteinelektrophorese zunutze macht. Weiterhin auf Präzipitationsverfahren, die darauf basieren, daß apo-B-haltige Lipoproteine (VLDL und LDL) durch Polyanionen und divalente Kationen von nicht-apo-B-haltigen Lipoproteinen durch Ausfällung der erstgenannten getrennt bestimmt werden können. Bei dem Apo-B-Protein handelt es sich um den hauptsächlichen Proteinanteil der low density bzw. β-Lipoproteine als auch der VLDL bzw. prä-β- Lipoproteine und Chylomikronen. Das letztgenannte Vefahren (Präzipitationstechniken) war bisher nicht geeignet, VLDL von LDL zu trennen. Die beiden erstgenannten Verfahren Ultrazentrifugation Havel, R.J., Eder H.A. und Bragdon J.H.: The Distribution and Chemical Composition of Ultracentrifugally Separated Lipoproteins in Human Serum, J.Clin.Invest. 34, 1345, 1955 und Elektrophorese Wieland H. und Seidel D.: Fortschritte in der Analytik des Lipoproteinmusters, Inn.Med. 5, 290 - 300,1978.. hatten bei der Anwendung größerer Routineserien den Nachteil, daß sie entweder zu kostspielig und zeitaufwendig oder nicht automatisierbar waren. Zusätzlich war eine direkte Messung des low density Cholesterins nur nach Isolierung der Fraktionen, üblicherweise unter Verwendung der Ultrazentrifuge, möglich. Die rechnerische Ermittlung des low density Cholesterinanteils über die Elektrophorese setzt gewisse Prämissen der Proteinlipidzusammensetzung voraus. Gleiches gilt für die Verfahren, die sich die Präzipitationstechniken zunutze machen. Die Präzipitationstechniken der üblichen Weise haben zusätzlich den Nachteil, daß sie das Lipoproteinspektrum nur in zwei Hauptfraktionen (apo-B-haltige und nicht-apo-B-haltige) auftrennen, also keine Unterscheidung oder Trennung von VLDL und LDL möglich ist.

Therapeutische Bemühungen, die low density Lipoproteine effektiv zu senken, waren bisher allesamt unbefriedigend. Eine medikamentöse Beeinflussung der low density Lipoproteinkonzentration ist vor allen Dingen bei den genetischen Formen von Fettstoffwechselstörungen äußerst schwierig und in der Regel unbefriedigend. Bei den bisher medikamentös erzielten Effekten konnten der therapeutische Erfolg, das Atheroskleroserisiko zu senken, nicht sicher nachgewiesen werden. Chirurgische Eingriffe, die im wesentlichen auf dem Anlegen abnormer Blutkreislaufverhältnisse oder auf Verlegung größerer Teile des Darms beruhen, haben zwar deutliche therapeutische Erfolge gebracht, können allerdings wegen der zu erwartenden starken Nebenwirkungen nicht als allgemein vertretbar angesehen werden. Solch drastische therapeutische Manipulationen haben allerdings gezeigt, daß bei einer ausreichenden Senkung der low density Lipoproteine atherosklerotische Prozesse nicht nur zum Stillstand gebracht werden können, sondern bereits vorhandene atherosklerotische Gefäßveränderungen rückbildungsfähig sind.

Der Versuch, low density Lipoproteine "mechanisch" aus dem Blut zu eliminieren, wurde bisher im wesentlichen auf zwei Wegen beschritten: Durch die Behandlung der befallenen Patienten mit einer sog. Plasmapherese, einem Verfahren, bei dem es zu einem kompletten Austausch des gesamten Blutwassers kommt. Dieses Verfahren hat dazu geführt, den low density Lipoproteingehalt der befallenen Patienten zwar zu senken, gleichzeitig werden aber jene Lipoproteine, die der Atherosklerose entgegenwirken (high density Lipoproteine) ebenso aus dem Blut eliminiert, ein insgesamt unerwünschter therapeutischer Effekt. Hinzu kommt, daß beim Plasmaaustausch alle anderen Proteine des Plasmas, eingeschlossen Gerinnungsfaktoren, Globuline und Hormone, miteliminiert werden. Dennoch hat sich dieses Verfahren bisher und für bestimmte Fälle von Hyper-$\beta$-lipoproteinämie als durchaus brauchbar erwiesen, wenngleich die Suche nach einer selektiven Eliminierung der low density Lipoproteine aus dem Plasma nach wie vor vorrangig ist. Ein erster Versuche der Elimination von low density Lipoproteinen aus dem Blut wurde mit Hilfe von spezifischen Antikörpern, die an eine Matrix gekoppelt waren, unternommen. Die Antikörper wurden hierzu durch Immunisierung von Schafen oder Kaninchen gewonnen. Obgleich mit solchen Systemen (Habenicht, A. Inaugural-Dissertation, 1978, Heidelberg, Md. Fakultät,; Stoffel W. u. Demant, Th. Selective Removal of Apolipoprotein B-Containing Serum Lipoproteins - from Blood Plasma, Proc.Nat.Acad.Sci. USA 78, 611-615, 1981 eine drastische Senkung aller apo-B-haltigen Lipoproteine erzielt wurde, hat dieses Verfahren den Nachteil, daß während der therapeutischen Anwendung die in einem Tier erzeugten Antikörper zwar in geringen Mengen, aber nicht vermeidbar, in die Zirkulation des behandelnden Patienten gelangen. Dieses muß auf lange Sicht gesehen, immunologische Probleme bei der zu behandelnden Person auslösen. Dieser erhebliche Einwand gegen ein solches Verfahren ist umso gewichtiger als die Behandlung von Fettstoffwechselstörungen eine Lebzeitbehandlung sein muß, soll sie effektiv und nachhaltig wirken. Es ist zu fordern, daß gerade bei den familiären Formen die Therapie bereits im Jugendalter einsetzen muß, um den Prozeß der sich frühzeitig entwickelnden atherosklerotischen Gefäßerkrankung aufzuhalten oder meßbar zu verlangsamen. Ein weiterer Nachteil des Einsatzes immobilisierter Antikörper gegen Apo-B ist der der unspezifischen Elimination aller apo-B-haltigen Lipoproteine. Dieses Verfahren ist also nicht geeignet low density Lipoproteine als die einzigen atherogenen Lipoproteine selektiv zu eliminieren. Ein wesentlicher Nachteil der gleichzeitigen Entfernung der VLDL ist die dadurch bedingte Stimulation der Triglyceridund Cholesterinsynthese in der Leber.

Ein drittes Verfahren low density Lipoproteine aus dem Blut zu entfernen basierte auf der selektiven Absorption von $\beta$- und prä-$\beta$-Lipoproteinen aus dem Vollblut an an Agaroseperlchen gekoppelte sulfatierte Polysaccharide, z.B. Heparin oder Dextransulfat gemäss US-PS 4 103 685. Ein aus der PS ersichtlicher Nachteil ist die geringe Kapazität dieses Verfahrens zur Elimination der Lipoproteine sowie die Tatsache, daß nicht nur low density Lipoproteine, sondern auch very low density Lipoproteine mit entfernt werden. Cholesterinsenkungen sind nach dem Verfahren der US-PS 4 103 685 nur für in vitro-Versuche beschrieben. Die Cholesterinsenkung erscheint, wie aus der begleitenden Senkun der Phospholipide und Triglyceride hervorgeht, eher durch eine Verdünnung des Blutes des Patienten hervorgerufen worden zu sein als durch selektive Elimination.

Die in der US-PS4 215993 beschriebene Verfahrensweise, Lipoproteine allgemein in ihrem isoelektrischen Punkt zu fällen, bezieht sich im Unterschied zu dem vorliegenden erfindungsgemässen Verfahren auf die Verwendung von Natriumphosphorwolframat. Dieses Polyanion ist unphysiologisch und im Unterschied zum vorliegenden Verfahren daher ungeeignet,als therapeutisches Prinzip eingesetzt zu werden. Darüber hinaus gelingt es unter Verwendung von Phosphorwolframat nicht, low density Lipoproteine selektiv zu eliminieren oder zu bestimmen, also die Hauptzielsetzung der vorliegenden Erfindung wird nicht erreicht. Daß dieses nicht gelingt, wurde in der US-PS 4 125 993 u.a. dadurch aufgezeigt, daß man empfiehlt, die low density Lipoproteine nach einer Rechenformel (Friedewald-Formel) aus dem sog. HDL-Cholesterin unter zusätzlicher Berücksichtigung des Gesamtcholesterins und der Triglyceride zu errechnen. Im wesentlichen unterscheidet sich die isoelektrische Punktfällung unter Verwendung von Phosphorwolframsäure, wie in der US-PS 4 125 993 angegeben, in ihrem Resultat nicht von der üblichen Polyanionpräzipitation mit Verwendung zweiwertiger Kationen. Eine komplette Fällung von VLDL und LDL in dem genannten Verfahren wird nur erreicht, wenn Polymere, wie z.B. Polyvinylpyrolidon oder Polyäthylenglykol genau definierten Molekulargewichts der Präzipitationslösung zugesetzt werden. Daß dies in einem extrakorporalen System nicht zu verwirklichen ist, steht außer Frage.

Im übrigen ist darauf hinzuweisen, daß in der US-PS 4 125 993 irreführenderweise die Gesamtheit von $\beta$-Lipoproteinen, prä-$\beta$-Lipoproteinen und Chylomikronen als low density Lipoproteine bezeichnet wird. Da erklärtes Ziel der US-PS 4 125 993 ist, high density Lipoproteine im Serum zu bestimmen, muß Wert darauf gelegt werden, daß die neben high density Lipoproteinen noch vorkommenden VLDL, Chylomikronen und LDL durch dieses Präzipitationsverfahren eliminiert werden. Eine spezifische Präzipitation der wirklichen low density Lipoproteine oder $\beta$-Lipoproteine wurde weder beabsichtigt noch erreicht.

Die DE-OS 20 13 644 betrifft ein Verfahren und Reagens zur $\beta$-Lipoprotein-Bestimmung. Es wird ausgeführt, daß neben Heparin als Fällungsmittel für eine derartige Bestimmungsweise auch hochmolekulares Dextransulfat besonders gut geeignet ist. Das Dextransulfat hat ein Molekulargewicht von über 200 000. Das Reagens enthält das Dextransulfat in wäßriger Lösung.

Die vorliegende Erfindung stellt sich daher die Aufgabe, ein Verfahren zu entwickeln, das es gestattet, selektiv und mit hoher Kapazität low density Lipoproteine oder $\beta$-Lipoproteine aus dem Blut, d.h. dem Vollserum oder Bestandteilen des Blutes wie Plasma zu entfernen und dieses Prinzip für diagnostische Zwecke zu verwenden.

Gelöst wird die Aufgabe durch die Zurverfügungstellung eines dafür geeigneten Verfahrens.

Die Erfindung betrifft ein

Verfahren zur selektiven extrakorporalen Präzipitation von low density Lipoproteinen oder $\beta$-Lipoproteinen aus Vollserum oder Plasma zu diagnostischen Zwecken durch Zugabe von Polyanionen zu dem Vollserum oder Plasma in einem Puffer, dadurch gekennzeichnet, daß die Polyanionen Dextransulfat vom Molekularbereich 10 000 bis 2 000 000 oder Natriumphosphorwolframat sind und der gebildete $\beta$-Lipoprotein-Polyanion-Komplex am isoelektrischen Punkt bei einen pH-Wert von 5,05 bis 5,25 ausgefällt, anschließend abgetrennt und das Präzipitat bzw. das Filtrat gegebenenfalls weiter analysiert werden.

Das Verfahren beruht auf der absolut spezifischen Ausfällung der $\beta$-Lipoproteine nach Vernetzung mit den Polyanionen durch Ausfällung am isoelektrischen Punkt des Komplexes bei einem pH-Wert von 5,05 bis 5,25 Fällung im isoelektrischen Punkt durch Senkung des pH unter Verwendung von Puffern kann durch geeignete Filtration und pH-Angleichung nach der isoelektrischen Punktfällung leicht auf physiologische Verhältnisse hin korrigiert werden.

Das verbleibende Serum Kann durch Filtration von der Ausfällung abgetrennt werden und in den Blutkreislauf zurückgeführt.

Die Vorrichtung zur Durchführung des erfindungsgemäßen Verfahrens ist beschieben in EP-180 720 A2 und B1. EP-A- 0 180 720 ist sowie vorliegende Anmeldung eine Teilanmeldung zur Stammanmeldung EP-A- 00 74610. [Art.76.EPÖ]

Es wurde gefunden, daß sich als Polyanionen für die Lipoproteinpräzipitation Dextransulfat vom Molekularbereich 10 000 bis 2 000 000 sowie Natriumphosphorwolframat eignen.

Insbesondere wird bei dieser Ausführungsform des erfindungsgemässen Verfahrens der Komplex aus Polyanion, wie Dextransulfat oder Natriumphosphorwolframat, und $\beta$-Lipoproteinen bei einem pH-wert von 5,13 gefällt.

Das Polyanion, z.B das Dextransulfat, wird in einem Puffer gelöst und mit dem Vollserum oder Plasma vermischt. Der Puffer dient zur Einstellung und Stabilisierung der angegebenen pH-Werte. Alle Puffergemische, die eine Stabilität in dem angegebenen pH-Bereich sichern, sind prinzipiell für die Anwendung des Verfahrens verwendbar Als geeignete Puffer haben sich ein Phosphatpuffer oder ein Phosphat-Citrat-Puffer oder ein Lactatpuffer oder ein Acetatpuffer oder deren Gemische herausgestellt.

Das Verhältnis von Puffer zu Serum bzw. Plasma bzw. Blutvolumen kann zwischen 1 : 5 bis 1 000 : 1 liegen. Als günstigste Volumenverhältnisse für die selektive Elimination der low density-Lipoproteine aus dem Blut für diagnostische Zwecke hat sich ein Verhältnis zwischen 2 bis 10 : 1 Puffer zu Serum ergeben.

Die Polyanionkonzentration ist kein besonders kritischer Punkt. Günstigerweise wird man jedoch auf Polyanionkonzentrationen zurückgreifen, die nahezu komplett durch die $\beta$-Lipoproteine mit auspräzipitiert werden. Beispielsweise sollte eine gegebene Serummenge im günstigsten Falle mit 10 Vol-% einer 6-proz. Dextransulfat-Pufferlösung bzw. 40-proz. Natriumphosphorwolframat-Pufferlösung versetzt werden.

Beim Zusammenbringen von z.B. einer Mischung von gleichen Teilen des Vollserums oder Plasmas mit einem Phosphat-Citrat-Puffer, in dem das Polyanion gelöst ist, bei dem sich einstellenden pH-Wert von etwa 5,13, erfolgt die Ausfällung sofort und vollständig und ist spezifisch.

Das bedeutet, daß nur die $\beta$-Lipoproteine oder low density-Lipoproteine ausgefällt werden. Die ausgefällten $\beta$-Lipoproteine können beispielsweise durch einen Membranfilter der Porengröße von ca 0,2 bis 2 $\mu$m bzw. durch einfache Zentrifugation in einem Continuous-Flow-Verfahren vom verbleibenden Serum abgetrennt werden.

Bei der Diagnostik kann sowohl der von $\beta$-Lipoproteinen befreite Serumrückstand als auch der ausgefällte Komplex von $\beta$-Lipoproteinen und Polyanion weiteren Untersuchungen unterzogen werden. Die Spezifität dieser Ausführungsform des erfindunosgemässen Verfahrens wurde sowohl mit quantitativen immunologischen Techniken sowie der Ultrazentrifugation als auch quantitativen Lipoproteinelektrophorese überprüft. Die hier beschriebene spezifische pH-Fällung von low density Lipoproteinen führt zu einer kompletten Präzipitation dieser $\beta$-Lipoproteinklasse. Im verbleibenden Filtrat finden sich lediglich HDL und VLDL an Lipoproteinen.

Die selektive Elimination von LDL aus dem Plasma in vitro ermöglicht eine Bestimmung dieser Fraktion entweder aus Differenzberechnung zum Gesamtcholesterin und dem verbleibenden Filtrat oder durch die direkte Cholesterinbestimmung am Präzipitat bzw. durch Trübungsmessung des entstehenden Präzipitats. Diese direkte Bestimmung des LDL-bzw. $\beta$-Cholesterins ist neu und bisher in keinem konkurrierenden Verfahren beschrieben.

Diese Ausführungsform des erfindungsgemässen Verfahrens wurde sowohl mit der präparativen Ultrazentrifuge als auch mit der quantitativen Lipoproteinelektrophorese verglichen und hat mit beiden Verfahren Korrelationskoeffizienten von über 0.98 in einer Serie von über 100 Patientenseren ergeben.Die Spezifität dieser Ausführungsform des erfindungsgemässen Verfahrens ist der Ultrazentrifuge deutlich überlegen. Gegenüber der Elektrophorese hat diese Ausführungsform des Verfahrens den Vorteil der Möglichkeit einer direkten Cholesterinbestimmung der β-Lipoproteine.Diese Ausführungsform des erfindungsgem. Verfahrens lässt sich leicht sowohl in einem Diskretautomaten als auch in einem Continuous-Flow-Automaten als mechanisierte Methode betreiben. Die Präzision in der Serie wie auch von Tag zu Tag liegt unter 2 %.

Die Erfindung wird weiter in den folgenden Ausführungsbeispielen erläutert.

1. Plasma wird mit einer gleichen Menge eines Citrat-, Phosphat- oder Acetat-Puffers (0,05 M, pH 4,15) gemischt. Nach Hinzufügen von ca. 10 Vol.-% einer 6-proz. Dextransulfat-Pufferlösung bildet sich ein gelbweißer Niederschlag, der aus den ausgefällten β-Lipoproteinen und hauptsächlich Fibrinogen besteht.

2. 100 $\mu$l Serum werden mit 1 ml einer Lösung gemischt, die folgendermaßen zusammengesetzt ist: 0,0641 M Na-Citrat (pH 5,04), 1 Vol.-% einer 40-proz. Natriumphosphorwolframat-Pufferlösung. Das Gemisch wird 10 min in einer Eppendorf-Zentrifuge zentrifugiert. Der Cholesterin-Gehalt des Überstandes entspricht dem Cholesterin-Gehalt von VLDL und HDL. Die LDL sind ausgefällt worden und lassen sich aus der Differenz Vollserumcholesterin - (VLDL- + HDL-Cholesterin) errechnen.

## Patentansprüche

1. Verfahren zur selektiven extrakorporalen Präzipitation von low density Lipoproteinen oder β-Lipoproteinen aus Vollserum oder Plasma zu diagnostischen Zwecken durch Zugabe von Polyanionen zu dem Vollserum oder Plasma in einem Puffer, dadurch gekennzeichnet, daß die Polyanionen Dextransulfat vom Molekularbereich 10 000 bis 2 000 000 oder Natriumphosphorwolframat sind und der gebildete β-Lipoprotein-Polyanion-Komplex am isoelektrischen Punkt bei einem pH-Wert von 5,05 bis 5,25 ausgefällt, anschließend abgetrennt und das Präzipitat bzw. das Filtrat gegebenenfalls weiter analysiert werden.

2. Verfahren nach Anspruch 1 ,dadurch gekennzeichnet, daß die Ausfällung des β-Lipoprotein-Polyanion-Komplexes bei einem pH-Wert von 5,13 erfolgt.

3. Verfahren nach Ansprüchen 1-2, dadurch gekennzeichnet, daß als Puffer Phosphatpuffer, Citratpuffer, Lactatpuffer, Acetatpuffer oder deren Gemisch verwendet werden.

4. Verfahren nach Ansprüchen 1 - 3,dadurch gekennzeichnet, daß das Volumenverhältnis zwischen Puffer und Serum zwischen 2 bis 10 : 1 liegt.

5. Verfahren nach Ansprüchen 1 - 4,dadurch gekennzeichnet, daß nach dem Abtrennen des ausgefällten β-Lipoprotein-Polyanion-Komplexes das verbleibende Filtrat weiter analysiert wird oder das Präzipitat bzw. Teile dessen quantitativ bestimmt werden.

## Claims

1. A process for the selective extracorporeal precipitation of low density lipoproteins or β-lipoproteins from full serum or plasma for diagnostic purposes by addition of polyanions to the full serum or plasma in a buffer, characterized in that the polyanions are dextran sulfate of a molecular weight range of from 10,000 to 2,000,000 or sodium phosphotungstate and the β-lipoprotein-polyanion complex formed is precipitated at the isoelectric point at a pH value of from 5.05 to 5.25, is subsequently separated, and the precipitate and/or filtrate is/are optionally further analyzed.

2. The process according to claim 1, characterized in that the precipitation of the β-lipoprotein-polyanion complex is effected at a pH value of 5.13.

3. The process according to claims 1 to 2, characterized in that phosphate buffers, citrate buffers, lactate buffers, acetate buffers or a mixture thereof are used as the buffer.

4. The process according to claims 1 to 3, characterized in that the volume ratio of buffer to serum is between from 2 to 10 : 1.

5. The process according to claims 1 to 4, characterized in that after the separation of the precipitated β-lipoprotein-polyanion complex the remaining filtrate is further analyzed or the precipitate or parts thereof are quantitatively determined.

## Revendications

1. Procédé pour la précipitation sélective extracorporelle de lipoprotéines ou de béta-lipoprotéines basse densité du sérum complet ou du plasma sanguin à des fins diagnostiques , par addition de polyanions au sérum complet ou au plasma dans un tampon , caractérisé en ce que les polyanions sont du sulfate de dextranne dont la gamme de poids moléculaire s'étend de 10 000 à 2 000 000 ou du phosphotungstate de sodium et l'on soumet le complexe de $\beta$-lipoprotéine-polyanion à précipitation au point isoélectrique à un pH allant de 5,05 à 5,25 , puis on le sépare et l'on soumet éventuellement à des analyses complémentaires le précipité ou le filtrat .

2. Procédé selon la revendication 1 , caractérisé en ce que la précipitation du complexe de béta-lipoprotéine/polyanion a lieu à un pH de 5,13 .

3. Procédé selon les revendications 1 et 2 , caractérisé en ce qu'on utilise comme tampon du tampon de phosphate , du tampon de citrate, du tampon de lactate , du tampon d'acétate ou leur mélange .

4. Procédé selon les revendications 1 à 3 , caractérisé en ce que le rapport en volume entre le tampon et le sérum se situe entre 2 et 10 : 1 .

5. Procédé selon les revendications 1 à 4 , , caractérisé en ce que , après la séparation du complexe précipité de beta-lipoprotéine/polyanion , on soumet le filtrat restant à des analyses supplémentaires ou bien on effectue des déterminations quantitatives du précipité ou de parties de celui-ci .